Europäisches Patentamt

European Patent Office

Office européen des brevets

(19)

(11) Veröffentlichungsnummer: **0 279 830 B1**

(12) **EUROPÄISCHE PATENTSCHRIFT**

(45) Veröffentlichungstag der Patentschrift:
16.10.91 Patentblatt 91/42

(51) Int. Cl.$^5$: **A61F 2/30, A61F 2/34**

(21) Anmeldenummer: 87905590.3

(22) Anmeldetag: 29.08.87

(86) Internationale Anmeldenummer:
PCT/DE87/00387

(87) Internationale Veröffentlichungsnummer:
WO 88/01491 10.03.88 Gazette 88/06

(54) **ENDOPROTHESE IN MODULBAUWEISE ZUM ERSATZ EINES BECKENTEILES IM HÜFTGELENKBEREICH.**

(30) Priorität: 02.09.86 DE 3629799

(43) Veröffentlichungstag der Anmeldung:
31.08.88 Patentblatt 88/35

(45) Bekanntmachung des Hinweises auf die
Patenterteilung:
16.10.91 Patentblatt 91/42

(84) Benannte Vertragsstaaten:
AT BE CH FR GB IT LI LU NL SE

(56) Entgegenhaltungen:
DE-A- 3 027 063
DE-A- 3 205 526
FR-A- 2 225 141
GB-A- 2 016 275

(73) Patentinhaber: S + G IMPLANTS GMBH
Grapengiesserstrasse 34
W-2400 Lübeck (DE)

(72) Erfinder: GRUNDEI, Hans
Gärtnergasse 4
W-2400 Lübeck (DE)
Erfinder: HIPP, Erwin
Ismainger Strasse 3e
W-8000 München (DE)
Erfinder: HEINHUBER, Bernd
Ismainger Strasse 3e
W-8000 München (DE)

**Beschreibung**

Die Erfindung betrifft eine Endoprothese in Modulbauweise zum Ersatz eines Beckenteiles im Bereich des Hüftgelenkes, bestehend aus einem Schalenteil zur Aufnahme der Hüftgelenkkugel und aus daran mittels einer Zapfen-Loch-Verbindung befestigten Anschlußelementen zur Fixation der Endoprothese im Schamund Darmbeinbereich.

In der deutschen Auslegeschrift 28 09 556 ist eine Beckenteilprothese offenbart, die nach dem Modulprinzip konstruiert ist. Sie besteht aus einem im wesentlichen quaderförmigen Hauptkörper mit einem Aufnahmeraum für die Hüftgelenkpfanne und mit mehreren, mit Löchern versehenen Anschlußflächen und aus mehreren stiftförmigen Verbindungselementen, die einerseits in den genannten Löchern befestigt sind und andererseits mit dem verbleibenden Beckenknochenmaterial des Patienten verbunden werden.

Der Hauptkörper dieser Endoprothese ist ein relativ großer Quaderkörper, da er die mehreren Löcher für die stiftförmigen Verbindungselemente aufweisen muß, um diese Elemente je nach Gegebenheit beim Patienten in einem ausgewählten Loch befestigen zu können. Außerdem werden .... die in die Löcher eingesetzten Verbindungselemente mittels Schrauben an dem Hauptkörper befestigt, wofür zusätzliche Gewindelöcher im Hauptkörper vorgesehen sein müssen. Obwohl mit diesen Prothesenteilen eine in ihrer Gestalt dem jeweiligen Bedarf entsprechend den Gegebenheiten beim Patienten angepaßte Gesamtprothese zusammengestellt werden kann, ist diese jedoch beim Implantieren in den Patienten umständlicher und zeitraubender zu handhaben, weil die Verbindungselemente mit dem Hauptkörper im implantierten Zustand zum Verschrauben nicht so gut zugänglich sind, wenn eine optimale Lage der Gesamtprothese erhalten werden soll. Werden andererseits die ausgewählten Prothesenteile vor der Implantierung montiert, kann eine optimale Lage der Prothesenteile zueinander und damit eine optimale Funktion der Gesamtprothese im Körper des Patienten nicht immer gewährleistet werden, weil der Hauptkörper wenigstens mit drei Verbindungselementen am Beckenknochenmaterial verankert wird wobei nicht sicher ist, ob jedes Verbindungselement sicher im Beckenknochenmaterial wegen dessen ungüstigen Aufbaus nach der Knochenresektion verankert werden kann. Außerdem bedeuten die Gewindelöcher und die hierfür zu verwendenden Befestigungsschrauben einen zusätzlichen Fertigungsaufwand bei der Herstellung der Prothese.

Die Aufgabe der Erfindung besteht in der Verbesserung einer Endoprothese der einleitend angeführten Art, die neben der Möglichkeit, den anatomischen Gegebenheiten des erkrankten Hüftgelenkbereichs eines Patienten individuell angepaßt und wirtschaftlich hergestellt werden zu können, zusätzlich gewährleistet, daß die Prothese schnell und einfach sowie mit dem Ergebnis einer optimalen Funktion im implantierten Zustand implantiert werden kann.

Die Lösung der Aufgabe geht von der einleitend angeführten Endoprothese aus und kennzeichnet sich weiter dadurch, daß der Schalenteil zwei angeformte Ansätze mit jeweils einer Aufnahmebohrung aufweist, daß die Anschlußelemente mit ihrem Zapfen mittels einer Konus-Verbindung fest in den Aufnahmebohrungen der Ansätze angeordnet sind und daß die Anschlußelemente Löcher zu ihrer Schraubbefestigung an den entsprechenden Knochenbereichen aufweisen.

In bevorzugter Ausgestaltung der erfindungsgemäßen Endoprothese weisen die metallenen Anschlußelemente räumlich gekrümmte Plattenteile mit den Löchern zur Schraubbefestigung an den entsprechenden Knochenbereichen auf. Die knochenseitige Materialschicht des Schalenteiles wie auch der Anschlußelemente können zusätzlich als offenzellige Struktur zum Einwachsen von Knochenmaterial ausgebildet sein.

Die erfindungsgemäßen Endoprothese, deren Teile zwecks Einsetzung einer anatomisch individuell angepaßten Prothese in unterschiedlicher Größe vorliegen, kann schnell und einfach implantiert werden, weil die endgültige Festsetzung der Anschlußelemente, wenn diese an dem verbliebenen Beckenknochenmaterial lagerichtig befestigt sind, an dem kompakten Schalenteil durch den selbsthemmenden Konussitz ohne langwierige Montagetätigkeiten erfolgen kann und jeweils durch einen kurzen Schlag durchführbar ist. Ferner können die Prothesenteile aufgrund von nur zwei Anschlußelementen wesentlich leichter lagemäßig optimal implantiert werden, da dies eine leichtere Ausrichtung des Schalenteiles ermöglicht, so daß eine optimale Funktion der implantierten Prothese gewährleistet ist. Außerdem kann die vorgeschlagene Prothese wirtschaftlicher hergestellt werden, da zusätzliche Gewindelöcher mit zugehörigen Befestigungsschrauben in dem Schalenteil zur Befestigung der Anschlußelemente entfallen.

Die Erfindung ist nachstehend anhand eines in den anliegenden Zeichnungen dargestellten Ausführungsbeispieles näher erläutert. Es zeigen:

Figur 1 das Ausführungsbeispiel in Seitenansicht,

Figur 2 eine Ansicht auf ein erstes Prothesenteil gemäß dem Pfeil II in Figur 1,

Figur 3 eine Ansicht gemäß dem Pfeil III in Figur 2,

Figur 4 eine teilweise Schnittansicht gemäß der Linie IV-IV in Figur 3,

Figur 5 eine Seitenansicht auf ein zweites Prothesenteil des Ausführungsbeispieles,

Figur 6 eine Ansicht gemäß dem Pfeil VI in Figur 5,

Figur 7 eine weitere Ausführungsform des zweiten Prothesenteiles in Seitenansicht,

Figur 8 eine Seitenansicht eines dritten Prothesenteiles des Ausführungsbeispieles,

Figur 9 eine Ansicht gemäß dem Pfeil IX in Figur 8,

Figur 10

und 11 eine Alernative zur dritten Einzelheit in Vorder- bzw. Seitenansicht,

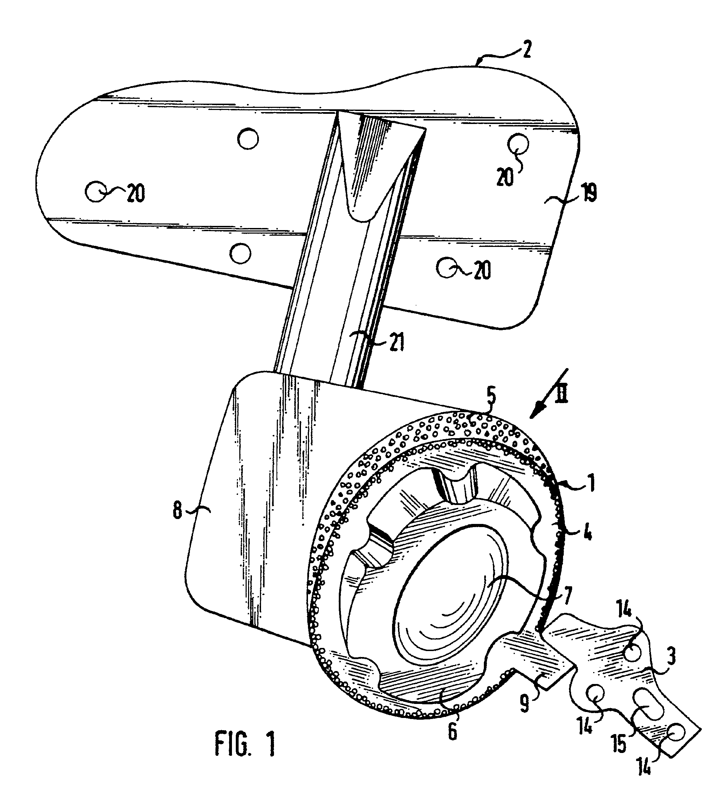

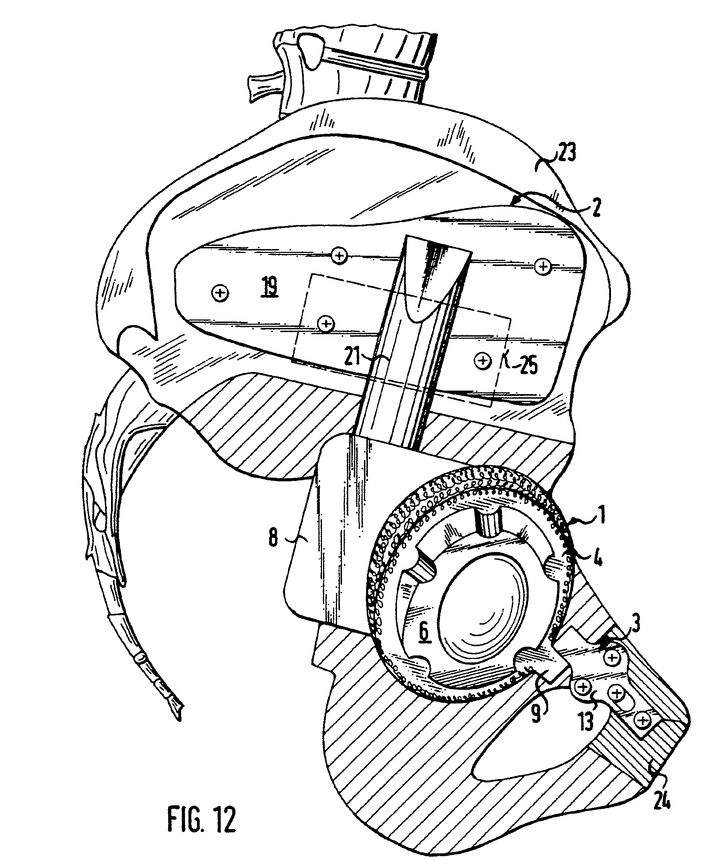

Figur 12 das Ausführungsbeispiel nach Figur 1 in implantiertem Zustand.

Gemäß Figur 1 besteht die Endoprothese zum Ersatz eines menschlichen Beckenteiles im Bereich des Hüftgelenkes aus mehreren, in Modulbauweise ausgeführten, miteinander verbindbaren Hauptbestandteilen, und zwar aus einem Schalenteil 1 zur Aufnahme einer nicht gezeigten künstlichen Hüftgelenkkugel, aus einem Anschlußelement 2 zur Fixierung am Darmbein und aus einem weiteren Anschlußelement 3 zur Fixierung am Schambein.

Der Schalenteilmodul 1 besteht im wesentlichen aus einer hohlen Halbkugel 4 aus Metall, z.B. aus hochlegiertem Stahl unter Verwendung von Chrom, Kobalt und Molybdän, bei der wenigstens die die Außenfläche der Halbkugel bildende Materialschicht in als offenzellige Struktur 5 zum Einwachsen von Knochenmaterial ausgebildet sein kann. In dem Schalenraum der Halbkugel ist eine ebenfalls halbkugelförmige Pfanne 6 aus Kunststoff, z.B. aus Polyäthylen, verankert, deren Hohlraum 7 eine künstliche Hüftgelenkkugel (nicht gezeigt) aufnimmt.

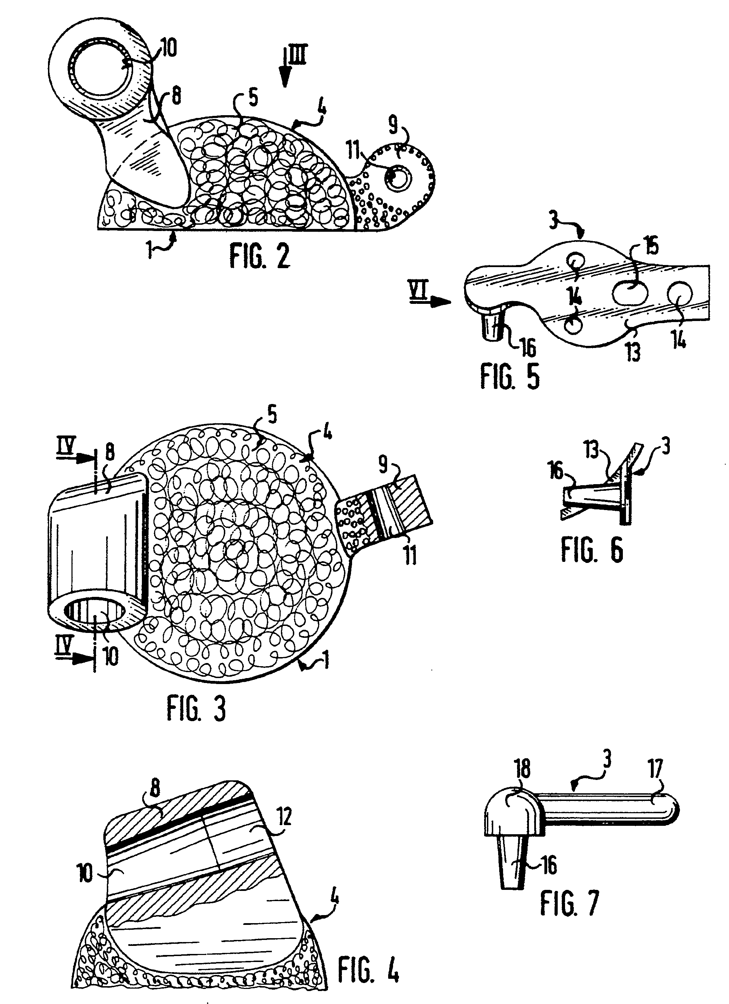

Der Schalenteil 1 weist an zwei sich etwa gegenüberliegenden Stellen seines Umfanges je einen angeformten Ansatz 8 und 9 auf, die zur Verbindung mit den Anschlußelementen 2 bzw. 3 ausgebildet sind. Hierzu zeigen die Figuren 2, 3 und 4 nähere Einzelheiten. Es ist daraus ersichtlich, daß die Ansätze 8, 9 zusammen mit der Halbkugel 4 aus einem einstückigen Gußteil bestehen, wobei die Ansätze nach Art eines Stehlagers geformt sind und je eine Aufnahmebohrung 10 und 11 zur Aufnahme des zugehörigen Anschlußelementes aufweisen. Die Aufnahmebohrungen 10,11 sind als konische Bohrungen ausgeführt, die mit konischen Zapfen der Anschlußelemente zusammenarbeiten, wie noch erläutert ist. Die konische Aufnahmebohrung 10 des größer ausgeführten, darmbeinseitigen Ansatzes 8 braucht nicht auf ihrer gesamten Länge konisch ausgeführt zu sein, sondern kann auch einen zylindrischen Abschnitt 12 aufweisen. Im übrigen braucht der Ansatz 8 nicht unbedingt im Bereich seiner Oberfläche mit einer offenzelligen Struktur versehen zu sein.

Die Figuren 5, 6 und 7 zeigen Ausführungsformen für schambeinseitige Anschlußelemente 3. Die Ausführungsform nach den Figuren 5 und 6 besteht aus einem länglichen, räumlich gekrümmten Plattenteil 13 mit mehreren Löchern 14,15 zur Schraubbefestigung an dem Schambein (Figur 10). Das Loch 15 ist als Langloch ausgebildet, um eine genaue Lage des Anschlußelementes 3 am Schambein einstellen zu können, bevor es endgültig festgeschraubt wird. An einem Ende des Plattenteiles 13 ist ein Konuszapfen 16 vorgesehen, der in die Aufnahmebohrung 11 des Ansatzes 9 des Schalenteiles 1 eingetrieben wird.

Das vorstehend beschriebene Anschlußelement 3 wie auch das Anschlußelement 2 nach Figur 7 bestehen aus dem gleichen Material wie der Schalenteil 1. Ferner kann wenigstens die knochenseitige Materialschicht dieser Teile zusätzlich als offenzellige Struktur zum Einwachsen von Knochenmaterial ausgebildet sein. Alternativ können diese Teile, ausgenommen ihre Zapfen 16, auch als durchgehend offenzellige Strukturkörper ausgebildet sein.

Das alternative Anschlußelement 3 nach Figur 7 weist einen fingerförmigen Fortsatz 17 von kreisförmigen Querschnitt mit einem hauptsächlich halbkugelförmigen Kopf 18 an seinem einen Ende auf, welch letzterer den bereits erwähnten, im rechten Winkel zu dem Fortsatz verlaufenden Zapfen 16 trägt. Während der fingerförmige Fortsatz 17 in eine entsprechende Ausnehmung des vorbereiteten Schambeins implantiert wird, greift der konische Zapfen 16 in die konische Aufnahmebohrung 11 des Schalenteilmoduls ein.

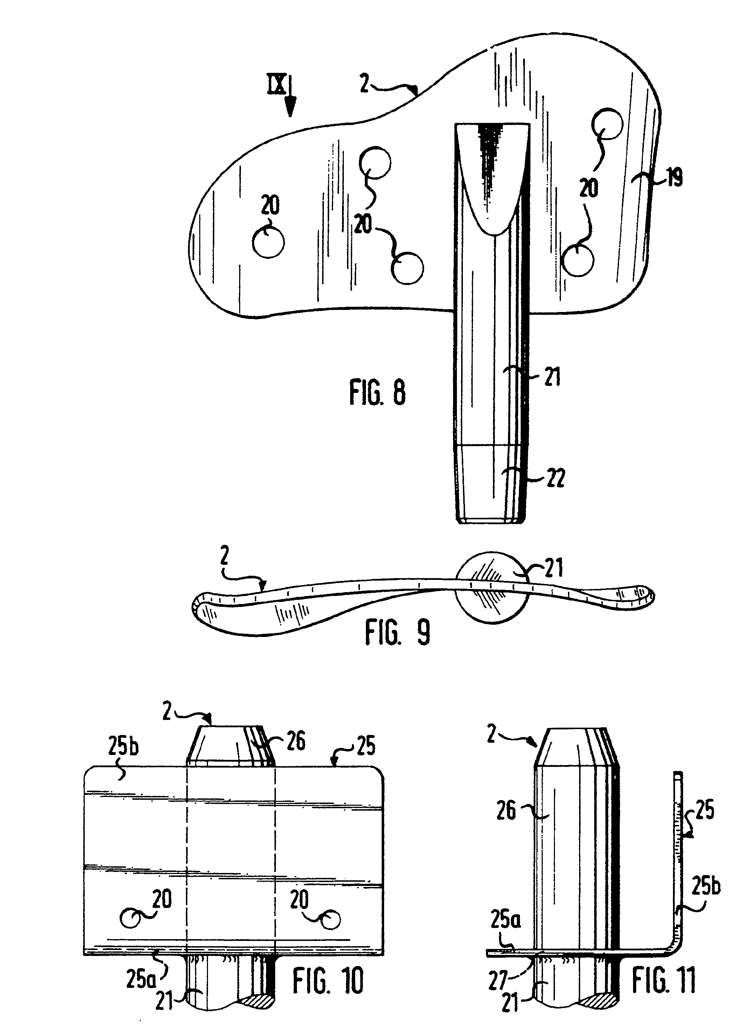

Das Anschlußelement 2 nach den Figuren 8 und 9, das aus dem gleichen Material hergestellt ist wie die anderen Teile 1 und 3, besteht ebenfalls aus einem etwas räumlich gekrümmten, etwa dreieckigen Plattenteil 19 mit mehreren Löchern 20 und aus einem Schaft 21, der an seinem freien Endabschnitt einen Konus 22 aufweist. Während der Plattenteil 19 mittels Schrauben an dem entsprechend vorbereiteten Darmbein befestigt wird, greift der Schaft 21 mit seinem Konus 22 in die konische Aufnahmebohrung 10 des anderen Ansatzes 8 des Schalenteiles 1 mit festem Reibschluß ein (Figur 1). Der Schaft 21 übergreift den Plattenteil 19 nur auf dessen Rückseite, wie Figur 8 zeigt, so daß also die später an dem Darmbein anliegende Seite des Plattenteiles 19 von Schaftmaterial frei ist. Auch dieser Plattenteil 19 kann wenigstens in seiner später dem Darmbein zugekehrten Randschicht aus einer offenzelligen Struktur bestehen; er kann aber auch durchgehend offenzellig strukturiert ausgebildet sein.

Eine Alternative zu dem Anschlußelement 2 ist in den Figuren 10 und 11 gezeigt. Das Plattenteil ist als L-förmiges Plattenteil 25 ausgebildet, wobei die beiden so .... entstandenen Schenkel 25a und 25b etwa im rechten Winkel zueinander verlaufen. Die Schenkel selbst können eben, wie gezeigt, oder auch räumlich gekrümmt sein. Auf der einen Seite des einen Schenkels 25a ist der weiter vorstehend bereits erwähnte Schaft 21 mit seinem Klemmzapfen vorgesehen, während auf der anderen Seite dieses Schenkels wenigstens ein vorspringendes Verankerungselement 26 vorgesehen ist. Es können auch mehrere, voneinander beabstandtete Verankerungselemente 26 vorgesehen sein. Wie die Figuren 10 und 11 zeigen, verläuft das Verankerungselement 26, das in eine entsprechend vorbereitete Ausnehmung des Darmbeins implantiert wird, und der vertikale Schenkel 25b des Plattenteiles 25 etwa parallel zueinander. Diese Teile können jedoch auch eine andere Zeigung zueinander aufweisen, wenn die anatomischen oder krankheitsbedingten Verhältnisse dies erfordern. Wenn dieses alternative Anschlußelement 2 implantiert ist, stützt sich das Darmbein auf dem horizontalen Schenkel 25a des Plattenteiles 25 ab, während der vertikale Schenkel 25b seitlich an dem Darmbein anliegt, wie es gestrichelt in Figur 12 angedeutet ist.

Eine einfache Ausführungsform des Verankerungselementes 26 besteht darin, daß der Schaft 21 nach oben verlängert ausgebildet ist. Der Schenkel 25a weist dann eine Durchbrechung 27 auf, durch die sich der Schaft 21 hindurcherstreckt, wobei der Schaftabschnitt oberhalb des Schenkels 25a das Verankerungselement bildet. In diesem Fall ist der Schaft mit dem Schenkel 25a verschweißt.

Alternativ können Plattenteil 25, Schaft 21 und Verankerungselement 26 auch als einstückiges Gußteil ausgebildet sein.

Im gezeigten Fall ist das Verankerungselement 26 zylindrisch ausgebildet; jedoch kann das oder jedes Verankerungselement 26 auch andere Querschnittsformen und andere Außenformen aufweisen. Ferner kann wenigstens die knochenseitige Randschicht des oder jedes Verankerungselementes als offenzellige Struktur zum Einwachsen von Knochenmaterial ausgebildet sein, wobei dies auch bei dem L-förmigen Plattenteil 25 so sein kann. Alternativ kann auch dieses Verankerungselement abgesehen von dem Schaft 21, durchgehend als offenzelliger Strukturkörper ausgebildet sein. Schließlich ist auch dieses Plattenteil 25 mit mehreren Löchern 20 zu seiner Befestigung am Darmbein versehen.

Figur 12 zeigt eine implantierte Endoprothese der vorstehend erläuterten Art. Nachdem die erkrankten Knochenteile im Hüftgelenkbereich des Beckenknochenaufbaus gemäß dem schraffierten Bereich entfernt und die Anlageflächen für die Anschlußelemente 2 und 3, d.h. für deren Plattenteile 19 bzw. 13, am Darmbein 23 bzw. am Schambein 24 vorbereitet worden sind, wird die Endoprothese, deren einzelne Teile 1,2 und 3 durch vorher erfolgte Ermittlung ihrer individuellen Größe, Form und Stellung zueinander bereits miteinander verbunden sein können, mit den entsprechenden Beckenknochen 23, 24 verschraubt.

Es ist klar, daß die einzelnen Prothesenteile in ihrer Form und Größe variieren können, um entsprechend den anatomischen Verhältnissen des betreffenden Hüftgelenkbereiches die am besten passenden Prothesenteile und damit die entsprechende Endoprothese verwenden zu können.

## Patentansprüche

1. Endoprothese in Modulbauweise zum Ersatz eines Beckenteiles im Bereich des Hüfgelenkes, bestehend aus einem Schalenteil (1) zur Aufnahame der Hüftgelenkkugel und aus daran mittels einer Zapfen-Loch-Verbindung befestigten Anschlußelementen (2,3) zur Fixation der Endoprothese im Schambzw. Darmbeinbereich, dadurch gekennzeichnet, daß der Schalenteil (1) zwei angeformte Ansätze (8, 9) mit jeweils einer Aufnahmebohrung (10, 11) aufweist, daß die Anschlußelemente (2, 3) mit ihrem Zapfen (16; 21, 22) mittels einer Konus-Verbindung in den Aufnahmebohrungen (10, 11) der Ansätze (8, 9) angeordnet sind und daß die Anschlußelemente (2, 3) Löcher (20; 14, 15) zu ihrer Schraubbefestigung an den entsprechenden Knochenbereichen aufweisen.

2. Prothese nach Anspruch 1, dadurch gekennzeichnet, daß der Schalenteil (1) als metallene hohle Halbkugel ausgebildet ist und daß die Anschlußelemente (2, 3) aus Metall bestehen und räumlich gekrümmte Plattenteile (19; 13) mit den genannten Löchern (20; 14, 15) aufweisen.

3. Prothese nach Anspruch 2, dadurch gekennzeichnet, daß das Plattenteil des darmbeinseitigen Anschlußelementes (2) aus einem L-förmigen Plattenteil (25) besteht und daß die eine Seite des einen Schenkels (25a) mit dem den Klemmzapfen (22) aufweisenden Schaft (21) und die andere Seite mit wenigstens einem vorstehenden Verankerungselement (26) versehen ist.

4. Prothese nach einem vorangehenden Anspruch, dadurch gekennzeichnet, daß die im implantierten Zustand gegen das Knochenmaterial zur Anlage kommenden Materialschichten der Prothesenteile (1, 2, 3) als offenzellige Struktur zum Einwachsen von Knochenmaterial ausgebildet sind.

## Claims

1. Endoprosthesis of modular construction for the replacement of part of the pelvis in the region of the hip joint, consisting of a dish part (1) to receive the ball of the hip joint and of connecting elements (2,3), attached to the said dish part by a peg-hole connection, for fixing the endoprosthesis in the region of the pubic bone or ilium, characterized in that the dish part (1) has two extensions (8,9), each with a receptive bore (10,11), in that the connecting elements (2,3) with their pegs (16,21,22) are arranged in the receptive bores (10,11) of the extensions (8,9) by means of a conical connection, and in that the connecting elements (2,3) have holes (20,14,15) for their screw attachment to the corresponding bone regions.

2. Prosthesis according to claim 1, characterized in that the dish part (1) is formed as a hollow metal hemisphere and in that the connecting elements (2,3) are made of metal and have spatially curved plate parts (19,13) with the said holes (20,14,15).

3. Prosthesis according to claim 2, characterized in that the plate part of the connecting element (2) on the ilium side consists of an L-shaped plate part (25) and in that one side of one arm (25a) is provided with the shank (21) with the clamping peg (22) and the other side with at least one projecting anchoring element (26).

4. Prosthesis according to a preceding claim, characterized in that the layers of material of the prosthesis parts (1,2,3) coming to bear on bone material in the implanted condition are formed as open-cell structure to promote the growing-in of bone material.

## Revendications

1. Endoprothèse à structure modulaire destinée à remplacer une partie du bassin dans la région de l'articulation de la hanche, constituée par un élément de coque (1) destiné à recevoir la rotule de l'articulation de la hanche et par des éléments de raccordement (2,3) fixés à cet élément de coque au moyen d'une liaison à broche et trou, pour la fixation de l'endoprothèse au niveau du pubis ou de l'ilion, caractérisée en ce que l'élément de coque (1) comporte deux parties saillantes moulées (8,9) possédant chacune un perçage récepteur (10,11), que les éléments de raccordement (2,3) sont disposés par leurs broches (16;21,22), au moyen d'une liaison conique, dans les perçages récepteurs (10,11) des parties saillantes (8,9) et que les éléments de raccordement (2,3) possèdent des trous (20;14,15) pour leur fixation par vissage aux parties correspondantes de l'os.

2. Prothèse selon la revendication 1, caractérisée en ce que l'élément de coque (1) est réalisé sous la forme d'une demi-sphère métallique creuse et que les éléments de raccordement (2,3) sont réalisés en métal et possèdent des éléments de plaques (19;13) cintrés dans l'espace et possédant lesdits trous (20;14,15).

3. Prothèse selon la revendication 2, caractérisée en ce que l'élément de plaque de l'élément de raccordement (2) du côté de l'ilion est formé par un élément de plaque en forme de L (25) et qu'un côté d'une branche (25a) comporte la tige (21), qui possède la broche de blocage (22), et que l'autre côté possède au moins un élément d'ancrage saillant (26).

4. Prothèse selon l'une des revendications précédentes, caractérisée en ce que les couches de matériau, qui, à l'état implanté, s'appliquent contre la masse osseuse des éléments (1,2,3) de la prothèse sont réalisées sous la forme d'une structure à cellules ouvertes permettant l'implantation de la masse osseuse.

FIG. 1

FIG. 2

FIG. 5

FIG. 3

FIG. 6

FIG. 4

FIG. 7

IX↓

2

20

20

20

19

20

21

22

FIG. 8

2

21

2

FIG. 9

2

26

25

25b

20

20

21

25a

FIG. 10

2

26

25

25b

25a

27

21

FIG. 11

FIG. 12